# EUROPÄISCHE PATENTSCHRIFT

(11) **EP 0 669 354 B1**
(45) Veröffentlichungstag und Bekanntmachung des Hinweises auf die Patenterteilung: **17.05.2000**
(21) Anmeldenummer: 94120893.6
(22) Anmeldetag: 29.12.1994
(51) Int. Cl.: C08G 18/79, C08G 18/80, C07D 229/00

(54) **Verfahren zur Herstellung von uretdiongruppenhaltigen Polyadditionsprodukten und deren Verwendung in Polyurethan-Lacksystemen**
Process for the preparation of polyaddition products containing uretdione groups and their use in polyurethane-lacquer systems
Procédé de préparation de produits de polyaddition contenant des groupes uretdione et leur utilisation dans des systèmes de vernis de polyuréthane

(30) Priorität: 28.02.1994 DE 4406445
(43) Veröffentlichungstag der Anmeldung: 30.08.1995
(73) Patentinhaber: Degussa-Hüls Aktiengesellschaft, 60287 Frankfurt am Main (DE)
(72) Erfinder: Gras, Rainer, Dr., D-44879 Bochum (DE); Brandt, Siegfried, Dr., D-45721 Haltern (DE); Behrendt, Klaus, D-44628 Herne (DE); Herda, Silvia, D-44623 Herne (DE)

(56) Entgegenhaltungen:
- EP-A- 0 033 927
- EP-A- 0 045 996
- EP-A- 0 519 734
- BE-A- 571 279
- FR-A- 2 298 566

## Beschreibung

Die Erfindung betrifft ein neues, einfaches Verfahren zur Herstellung von uretdiongruppenhaltigen Polyisocyanaten sowie ihre Verwendung in licht- und wetterstabilen Polyurethan (PUR)-Lacksystemen, insbesondere in PUR-Pulverlacken.

In der DE-OS 30 30 572 werden ein Verfahren zur Herstellung von uretdiongruppenhaltigen Polyadditionsprodukten sowie die danach hergestellten Produkte vorgestellt. Dabei handelt es sich um Reaktionsprodukte aus dem isocyanuratfreien Uretdion (UD) des 3-Isocyanatomethyl-3.5.5-trimethylcyclohexylisocyanats (Isophorondiisocyanat, IPDI) - herstellbar gemäß der DE-OS 30 30 513 oder DE-OS 37 39 549 - und Diolen sowie ggf. Monoalkoholen oder -aminen. Die Reaktion kann in Substanz oder auch in Gegenwart geeigneter Lösemittel erfolgen. In der Praxis sind bisher marktrelevante Verkaufsmengen dieser Vernetzerklasse ausschließlich in geeignetem Lösemittel unter schonenden Bedingungen, bei ca. 60 °C, produziert worden, um die thermische Ringspaltung während der Synthese zu vermeiden. Die Herstellung in Substanz ist bisher über den Labormaßstab nicht hinaus gekommen, da sich in Abhängigkeit von der Vernetzer-Molmasse im Laufe der Reaktion ein hohes Viskositätsniveau aufbaut; Temperaturerhöhung als Maßnahme zur Viskositätsregulierung ist, wie in der DE-OS 30 30 572 angeführt, limitiert. Die technische Realisierung der lösemittelfreien Herstellung uretdiongruppenhaltiger Polyadditionsprodukte ist bisher nicht gelungen, obwohl es an entsprechenden Versuchen nicht gefehlt hat, diese Problematik zu lösen. Die Produktion im Lösemittel hat nicht nur den Nachteil, daß das oder die Lösemittel nachträglich wieder entfernt werden müssen, sondern es sind auch lange Reaktionszeiten sowie aufwendige, spezielle Technologien zur Lösemittelentfernung erforderlich; das Befreien der Reaktionsprodukte vom Lösemittel erfolgt unter Vakuum bei ca. 120 °C unter Einsatz von Dünnschichtern, Filmtrudern oder Extrudern. Dadurch werden hohe Verfahrenskosten verursacht.

Aufgabe der vorliegenden Erfindung ist es daher, ein weniger aufwendiges, einfaches Verfahren zur Herstellung uretdiongruppenhaltiger Polyisocyanate für die Formulierung von PUR-Lacksystemen zur Verfügung zu stellen, welches die genannten Nachteile nicht aufweist.

Überraschenderweise wurde gefunden, daß die Umsetzung von Polyisocyanat-Uretdion mit Diolen sowie ggf. mit Monoalkoholen oder -aminen in Substanz, also lösemittelfrei, kontinuierlich in einem Intensiv-Mischer durchgeführt werden kann, wenn bestimmte Verfahrensbedingungen eingehalten werden.

Gegenstand der Erfindung ist daher ein Verfahren zur Herstellung von uretdiongruppenhaltigen Polyadditionsprodukten auf Basis von Polyisocyanat-Uretdion mit mindestens zwei freien Isocyanatgruppen, Diolen sowie ggf. Monoalkoholen oder -aminen, welches dadurch gekennzeichnet ist, daß die uretdiongruppenhaltigen Polyadditionsprodukte lösemittelfrei und kontinuierlich hergestellt werden.

Das Prinzip des Verfahrens besteht darin, daß die Umsetzungsprodukte kontinuierlich in einem Intensivkneter, einem Ein- oder Mehrschneckenkneter, insbesondere in einem Zweischneckenextruder kurzzeitig auf - für uretdiongruppenhaltige Polyisocyanate ungewöhnliche, für die lösemittelfreie Herstellung jedoch notwendige - hohe Temperaturen erhitzt werden können.

Überraschend war es, daß die Synthesetemperaturen auch mehr als 120 °C, bis 190 °C, betragen können. Diese Temperaturen liegen bereits deutlich im Rückspaltbereich für Uretdione, so daß dadurch hohe freie Isocyanatgehalte bzw. unkontrollierte Reaktionsverläufe zu erwarten wären. Das war jedoch nicht der Fall.
Die Temperaturen im Intensivkneter bzw. Zweischneckenextruder können bis 190 °C, vorzugsweise bis 180 °C, und insbesondere bis 170 °C betragen.

Weiterhin von prinzipieller Natur ist, daß die kurzzeitige thermische Belastung ausreicht, um die Reaktionspartner homogen zu mischen und dabei vollständig oder weitgehendst umzusetzen. Anschließend wird entsprechend der Gleichgewichtseinstellung gezielt abgekühlt und, falls erforderlich, der Umsatz vervollständigt.

Die Intensivkneter ermöglichen durch geeignete Bestückung der Mischkammern bzw. Zusammenstellung der Schneckengeometrie intensive rasche Durchmischung und hochviskose Produktströme bei gleichzeitigem intensiven Wärmeaustausch. Andererseits ist auch eine gleichmäßige Durchströmung in Längsrichtung mit möglichst einheitlicher Verweilzeit gewährleistet. Außerdem muß eine unterschiedliche Temperierung in den einzelnen Gerätegehäusen oder -abschnitten möglich sein.

Die Umsetzungsprodukte werden dem Intensivkneter in getrennten Produktströmen zudosiert, bei mehr als zwei Produktströmen können diese auch gebündelt zugeführt werden. Diole und/oder Monoalkohole/-amine und/oder Katalysatoren und/oder andere übliche Lack-Zuschlagsstoffe wie Verlaufmittel und Stabilisatoren können zu einem Produktstrom zusammengefaßt werden; ebenso Polyisocyanat-UD und solche, die gegenüber Isocyanatgruppen inert sind: Katalysatoren, und entsprechend obengenannte Lack-Zuschlagsstoffe.

Ferner kann die Eintrittsstelle der Produktströme in der Reihenfolge variabel und zeitlich versetzt gehandhabt werden.

Zur nachträglichen gezielten Abkühlung können eingesetzt werden: Rohrbündel, Rohrschlangen, Kühlwalzen, Luftförderer und speziell Transportbänder aus Metall, wobei Temperaturzonen einstellbar sein sollten. Ggf. kann die nachträgliche gezielte Abkühlung in dem Reaktionsteil integriert sein in Form einer mehrgehäusigen Ausführungsform wie bei Extrudern oder Conterna-Maschinen.

Die Konfektionierung wird je nach Viskosität des den Schneckennextruder und/oder die Nachreaktionszone verlassenden Produkts zunächst durch weitere Abkühlung mittels entsprechender vorgenannter Gerätschaften auf eine zur späteren Absackung/Silierung hinreichende Temperatur eingeleitet, wobei der Absackung eine Zerkleinerung vorgeschaltet ist. Während des Abkühlens kann an geeigneter Stelle eine Vorprägung des bevorzugt bandförmig anfallenden Produkts erfolgen, die eine nachfolgende Zerkleinerung in eine gewünschte Partikelgröße bzw. Granulatform mittels Walzenbrecher, Stiftmühle, Hammermühle oder ähnlichem vorbereitet und erleichtert sowie den Staubanfall vermindert. Bei Anwendung eines Kühlwalzenstuhls läßt sich diese Vorprägung damit kombinieren und der nachfolgend anfallende Staubanteil unmittelbar rückführen und wieder in das Produkt einarbeiten.

Überraschenderweise unterscheiden sich die in bekannter Weise in geeigneten Lösemitteln unter schonenden Bedingungen hergestellten Vernetzer im Hinblick auf die erfinderisch synthetisierten Vernetzer bezüglich der physikalischen und chemischen Kenndaten sowie der lacktechnischen Eigenschaften nur innerhalb der Fehlergrenzen.

Als Ausgangsverbindungen für die erfindungsgemäß synthetisierten Produkte können sowohl die Uretdione der Diisocyanate verwendet werden bzw. Uretdione, hergestellt aus zwei verschiedenen Diisocyanaten, sogenannte "gemischte" Dimerisate (Uretdione), als auch Gemische von Uretdionen. Die Diisocyanate im Sinne der vorliegenden Erfindung sind: aliphatische, (cyclo)aliphatische und araliphatische Diisocyanate, wie sie z. B. in Houben-Weyl, Methoden der organischen Chemie, Band 14/2, S 61 - 70 und dem Artikel von W. Siefken in Justus Liebigs Annalen der Chemie 562, S. 75 - 136, beschrieben werden, wie 1.4-Tetramethylendiisocyanat, 1.6-Hexamethylendiisocyanat (HDI), Methyl-pentamethylendiisocyanat, 2.2.4(2.4.4)-Trimethyl-1.6-hexamethylendiisocyanat (TMDI), Cyclohexan-1.3- und 1.4-diisocyanat, 3-Isocyanatomethyl-3.5.5-trimethylcyclohexylisocyanat [Isophorondiisocyanat (IPDI)], Hexahydroduroldi- isocyanat, 4.4'-Diisocyanatodicyclohexylmethan (HMDI), m- oder p-Tetramethylxylylendiisocyanat (TMXDI) sowie Hexahydroxylylendiisocyanat-1.4 und -1.3. Besonders geeignet ist das Uretdion des Isophorondiisocyanats. Die Herstellung der Diisocyanat-Uretdione erfolgt nach bekannten Methoden der Isocyanatchemie.

Zur Herstellung der uretdiongruppenhaltigen Polyadditionsprodukte werden Diole verwendet. Beispiele für derartige zweiwertige Alkohole sind: Ethylenglykol (E), Propylenglykol, wie 1,2- und 1,3-Propandiol, 2-Methylpropandiol-1.3 (MP), 2,2-Dimethylpropandiol-1.3 (NPG) Butandiol-1.4 (B), Diethylenglykol (DEG), Hexandiol (HD), Oktandiol 1,8, 3-Methylpentandiol (Pm), 2.2.4(2.4.4)-Trimethylhexandiol (TMH-d), Dodecandiol-1,12, Oktadecandiol-1,18, Hydroxypivalinsäureneopentylglykolester (Eg), trans- und cis-1,4-Cyclohexandimethanol (DMC).

Für die erfinderisch zu synthetisierenden uretdiongruppenhaltigen Polyadditionsprodukte liegt für die Polyisocyanat-Uretdion/Diol-Reaktion ein OH/NCO-Verhältnis von 0,5 bis 1,5 : 1 zugrunde, wobei ggf. die verbleibenden freien NCO-Gruppen total oder partiell mit Monoalkoholen oder -aminen umgesetzt werden.

Geeignete einwertige Alkohole sind: Methanol (M), Ethanol (Et), n- oder i-Propanol, n-Butanol, 2-Ethyl-hexanol (EH), n-Decanol, Cyclohexanol. Als Monoamine eignen sich: n-Propylamin, n-Butylamin, n-Hexylamin, Dibutylamin (DBA), Dicyclohexylamin.

Zur Beschleunigung der Isocyanatpolyadditionsreaktion können Katalysatoren eingesetzt werden. Geeignet sind organische Zinn-II- und Zinn-IV-verbindungen, wie Zinn-II-acetat, Zinn-II-octoat, Zinn-II-laurat, Dibutylzinndilaurat (DBTL), Dibutylzinndiacetat, Dibutylzinnmaleat oder Dioctylzinndiacetat oder Zinkoctoat sowie andere bekannte und in der Isocyanatchemie übliche Verbindungen. Die Katalysatorkonzentration liegt zwischen 0,01 und 2 Gew.-%, vorzugsweise zwischen 0,05 und 1 Gew.-% bezogen auf das Endprodukt.

Ein weiterer Gegenstand der vorliegenden Erfindung ist die Verwendung der erfindungsgemäß hergestellten uretdiongruppenhaltigen Polyadditionsprodukte zur Formulierung lagerstabiler, hitzehärtbarer PUR-Pulverlacke und lösemittelhaltiger Einkomponenten-PUR-Einbrennlacke.

Als Reaktionspartner für PUR-Pulverlacke kommen Verbindungen infrage, welche solche funktionellen Gruppen tragen, die sich mit Isocyanatgruppen während des Härtungsprozesses in Abhängigkeit von der Temperatur und Zeit umsetzen, z. B. Hydroxyl-, Carboxyl-, Mercapto-, Amino-, Urethan- und (Thio)-Harnstoffgruppen. Als Polymere können Polymerisate, Polykondensate und Polyadditionsverbindungen eingesetzt werden.

Bevorzugte Komponenten sind in erster Linie Polyether, Polythioether, Polyacetale, Polyesteramide, Epoxidharze mit Hydroxylgruppen im Molekül, Aminoplaste und ihre Modifizierungsprodukte mit polyfunktionellen Alkoholen, Polyazomethine, Polyurethane, Polysulfonamide, Melaminabkömmlinge, Celluloseester und -ether, teilweise verseifte Homo- und Copolymerisate von Vinylestern, insbesondere aber Polyester und Acrylatharze.

Die einzusetzenden hydroxylgruppenhaltigen Polyester haben eine OH-Funktionalität von 2,5 bis 5, bevorzugt von 3 bis 4,2, ein mittleres Molekulargewicht von 1 800 bis 5 000, bevorzugt von 2 300 bis 4 500, eine OH-Zahl von 25 bis 180 mg KOH/g, bevorzugt von 30 bis 140 mg KOH/g, eine Viskosität von < 80 000 mPa·s, bevorzugt von < 60 000 mPa·s, besonders bevorzugt von < 40 000 mPa·s bei 160 °C und einem Schmelzpunkt von ≥ 70 °C bis ≤ 120 °C, bevorzugt von 75 °C bis 100 °C.

Für die Herstellung von Polyestern bevorzugte Carbonsäuren können aliphatischer, cycloaliphatischer, aromatischer und/oder heterocyclischer Natur sein und gegebenenfalls durch Halogenatome substituiert und/oder ungesättigt sein. Als Beispiele hierfür seien genannt: Bernstein-, Adipin(As), Kork-, Azelain, Sebacin-, Phthal-, Terephthal- (Ts), Isophthal-(Is), Trimellit-, Pyromellit-, Tetrahydrophthal-, Hexahydrophthal-, Hexahydroterephthal-, Di- und Tetrachlorphthal-, Endomethylentetrahydrophthal-, Glutar-, Malein- und Fumarsäure bzw. - soweit zugänglich - deren Anhydride, Terephthalsäuredimethylester (DMT), Terephthalsäure-bis-glykolester, weiterhin cyclische Monocarbonsäuren wie Benzoesäure, p-tert.-Butylbenzoesäure oder Hexahydrobenzoesäure.

Als mehrwertige Alkohole kommen z. B. Ethylenglykol, Propylenglykol-1.2 und -1.3, Butylenglykol-1.4 und -2.3, Di-β-hydroxyethylbutandiol, Hexandiol-1.6, Octandiol-1.8, Neopentylglykol, Cyclohexandiol, Bis-(1.4-hydroxymethyl)-cyclohexan, 2.2-Bis-(4-hydroxycyclohexyl)-propan, 2.2-Bis-{4-(β-hydroxyethoxy)-phenylpropan, 2-Methyl-propandiol-1.3, 3-Methyl-pentandiol-1.5, 2.2.4(2.4.4)-Trimethylhexandiol-1.6, Glycerin, Trimethylolpropan, Trimethylolethan, Hexantriol-1.2.6, Butantriol-1.2.4, Tris-(β-hydroxyethyl)-isocyanurat, Pentaerythrit, Mannit und Sorbit sowie Diethylenglykol, Triethylenglykol, Tetraethylenglykol, Dipropylenglykol, Polypropylenglykole, Polybutylenglykole, Xylylenglykol und Hydroxypivalinsäureneopentylglykolester, infrage.

Auch Mono- und Polyester aus Lactonen, z. B. ε-Caprolacton oder Hydroxycarbonsäuren, z. B. Hydroxypivalinsäure, Ω-Hydroxydecansäure, Ω-Hydroxycapronsäure, können eingesetzt werden. Polyester aus den obengenannten Polycarbonsäuren bzw. deren Derivaten und Polyphenolen, wie Hydrochinon, Bisphenol-A, 4.4'-Dihydroxybiphenyl oder Bis-(4-hydroxyphenyl)-sulfon; Polyester der Kohlensäure, die aus Hydrochinon, Diphenylolpropan, p-Xylylenglykol, Ethylenglykol, Butandiol oder Hexandiol-1.6 und anderen Polyolen durch übliche Kondensationsreaktionen, z. B. mit Phosgen oder Diethyl- bzw. Diphenylcarbonat, oder aus cyclischen Carbonaten wie Glykolcarbonat oder Vinylidencarbonat, durch Polymerisation in bekannter Weise erhältlich sind; Polyester der Kieselsäure, Polyester der Phosphorsäure, z. B. aus Methan-, Ethan-, β-Chlorethan-, Benzol- oder Styrolphosphorsäure-, -phosphorsäurechchlorid oder -phosphorsäureester und Polyalkoholen oder Polyphenolen der obengenannten Art; Polyester der Borsäure; Polysiloxane, wie z. B. die durch Hydrolyse von Dialkyldichlorsilanen mit Wasser und nachfolgende Behandlung mit Polyalkoholen, die durch Anlagerung von Polysiloxandihydriden an Olefinen, wie Alkylalkohol oder Acrylsäure, erhältlichen Produkte.

Bevorzugte Polyester sind z. B. auch die Reaktionsprodukte von Polycarbonsäuren und Glycidylverbindungen, wie sie z. B. in der DE-OS 24 10 513, beschrieben sind.

Beispiele für Glycidylverbindungen, die verwendet werden können, sind Ester des 2.3-Epoxy-1-propanols mit monobasischen Säuren, die 4 bis 18 Kohlenstoffatome haben, wie Glycidylpalmitat, Glycidyllaurat und Gylcidylstearat, Alkylenoxide mit 4 bis 18 Kohlenstoffatomen, wie Butylenoxid und Glycidylether, wie Octylglycidylether.

Als Dicarbonsäuren können bei diesem Verfahren sämtliche unter II im folgenden aufgeführte Polycarbonsäuren verwendet werden, Monocarbonsäuren, welche beispielsweise unter III aufgeführt sind, können ebenfalls eingesetzt werden.

Bevorzugte Komponenten sind auch monomere Ester, z. B. Dicarbonsäure-bis-(hydroxy(alkohol)ester, Monocarbonsäureester von mehr als zweiwertigen Polyolen und Oligoester, die durch Kondensationsreaktionen aus in der Lackchemie üblichen Rohstoffen hergestellt werden können. Als solche sind z. B. anzusehen:
I. Alkohole mit 2 bis 24, vorzugsweise 2 bis 10 C-Atomen, und 2 bis 6 an nicht-aromatischen C-Atomen gebundenen OH-Gruppen, z. B. Ethylenglykol, Propylenglykol, Diethylenglykol, Dipropylenglykol, Butandiole, Neopentylglykol, Hexandiole, Hexantriole, Perhydrobisphenol, Dimethylolcyclohexan, Glycerin, Trimethylolethan, Trimethylolpropan, Pentaerythrit, Dipentaerythrit, Mannit;
II. Di- und Polycarbonsäuren mit 4 bis 36 C-Atomen und 2 bis 4 Carboxylgruppen sowie deren veresterungsfähige Derivate, wie Anhydride und Ester, z. B. Phthalsäure(anhydrid), Isophthalsäure, Terephthalsäure, Alkyltetrahydrophthalsäure, Endomethylentetrahydrophthalsäureanhydrid, Adipinsäure, Bernsteinsäure, Maleinsäure, Fumarsäure, Dimerfettsäuren, Trimellithsäure, Pyromellitsäure, Azelainsäure;
III. Monocarbonsäuren mit 6 bis 24 C-Atomen, z. B. Caprylsäure, 2-Ethylhexansäure, Benzoesäure, p-tert.-Butylbenzoesäure, Hexahydrobenzoesäure, Monocarbonsäuregemische natürlicher Öle und Fette, wie Cocosfettsäure, Sojaölfettsäure, Ricinenfettsäure, hydrierte und isomerisierte Fettsäuren, wie "Konjuvandol"-Fettsäure sowie deren Gemische, wobei die Fettsäuren auch als Glyceride einsetzbar sind und unter Umesterung und/oder Dehydratisierung umgesetzt werden können;
IV. einwertige Alkohole mit 1 bis 18 C-Atomen, z. B. Methanol, Ethanol, Isopropanol, Cyclohexanol, Benzylalkohol, Isodecanol, Nonanol, Octanol, Oleylalkohol.

Die Polyester können auf an sich bekannte Weise durch Kondensation in einer Inertgasatmosphäre bei Temperaturen von 100 bis 260 °C, vorzugsweise 130 bis 220 °C, in der Schmelze oder in azeotroper Fahrweise gewonnen werden, wie es z. B. in Methoden der Organischen Chemie (Houben-Weyl), Bd. 14/2, 1 - 5, 21 23, 40 44, Georg Thieme Verlag, Stuttgart, 1963 oder bei C. R. Martens, Alkyd Resins, 51 - 59, Reinhold Plastics Appl. Series, Reinhold Publishing Comp., New York, 1961, beschrieben ist.

Bevorzugte Acrylatharze, welche als OH-Komponente verwendet werden können, sind Homo- oder Copolymerisate, wobei z. B. folgende Monomere als Ausgangsprodukte gewählt werden können: Ester der Acrylsäure und Methacrylsäure mit zweiwertigen, gesättigten, aliphatischen Alkoholen mit 2 bis 4 C-Atomen, wie z. B. 2-Hydroxyethylacrylat, 2-Hydroxypropylacrylat, 4-Hydroxybutylacrylat und die entsprechenden Methacrylsäureester; Acrylsäure und Methacrylsäurealkylester mit 1 bis 18 C-Atomen in der Alkoholkomponente, wie z. B. Methacrylat, Ethylacrylat, Propylacrylat, Isopropylacrylat, n-Butylacrylat, tert.-Butylacrylat, 2-Ethylhexylacrylat, Stearylacrylat und die entsprechende Methacrylatsäureester; Acrylsäure- und Methacrylsäurecyclohexylester; Acrylnitril und Methacrylnitril, Acrylamid und Methacrylamid; N-Methoxymethyl(meth)acrylsäureamid.

Besonders bevorzugte Acrylatharze sind Copolymere aus
a. 0 bis 50 Gew.-% Ester der Acryl- oder Methacrylsäure mit zwei oder mehrwertigen Alkoholen, wie Butandiol-(1.4)-monoacrylat, Hydroxypropyl(meth)acrylat; ferner Vinylglykol, Vinylthioethanol, Allylalkohol, Butandiol-1.4-monovinylether;
b. 5 bis 95 Gew.-% Ester der Acrylsäure oder Methacrylsäure mit einwertigen Alkoholen, die 1 bis 12 Kohlenwasserstoffatome enthalten, wie z. B. Methylmethacrylat, Ethylacrylat, n-Butylacrylat oder 2-Ethylhexylacrylat;
c. 0 bis 50 Gew.-% aromatische Vinylverbindungen, wie Styrol, Methylstyrol oder Vinyltoluol;
d. 0 bis 20 Gew.-% andere Monomere mit funktionellen Gruppen, wie z. B. Acrylsäure, Methacrylsäure, Crotonsäure, Itaconsäure, Maleinsäure, Fumarsäure, Maleinsäureanhydrid, Maleinsäurehalbester, Acrylamid, Methacrylamid, Acrylnitril oder N-Methylol(meth)acrylamid sowie Glycidyl(meth)acrylat, wobei der Anteil der Gruppe a. und/oder b. mindestens 5 Gew.-% betragen.

Die Acrylatharze können nach den üblichen Methoden hergestellt werden, also durch Lösungs-, Suspensions-, Emulsions- oder Fällungspolymerisation; bevorzugt aber durch Substanzpolymerisation, die ihrerseits mittels UV-Licht initiiert werden kann.

Als weitere Polymerisationsinitiatoren werden die üblichen Peroxide oder Azoverbindungen, wie z. B. Dibenzoylperoxid, tert.-Butylperbenzoat oder Azodiisobutyronitril verwendet. Das Molekulargewicht kann z. B. mit Schwefelverbindungen, wie tert.-Dodecylmercaptan, geregelt werden.

Bevorzugte Polyether können z. B. durch Polyaddition von Epoxiden, wie Ethylenoxid, Propylenoxid, Butylenoxid, Trimethylenoxid, 3,3-Bis-(chlormethyl)-oxacyclobutan, Tetrahydrofuran, Styroloxid, dem Bis-(2.5)-epoxypropylether des Diphenylolpropans oder Epichlorhydrin mit sich selbst, z. B. in Gegenwart von BF₃, oder durch Anlagerung dieser Epoxide, gegebenenfalls im Gemisch oder nacheinander, an Startkomponenten mit reaktionsfähigen Wasserstoffatomen, wie Alkohole oder Amine, z. B. Wasser, Ethylenglykol, Propylenglykol-(1.3) oder -(1.2), Pentamethylenglykol, Hexandiol, Decamethylengylkol, Trimethylolpropan, 4.4'-Dihydroxydiphenylpropan, Anilin, Ammoniak, Ethanolamin, Ethylendiamin, Di(β-hydroxypropyl)-methylamin, Di(β-hydroxyethyl)-anilin, Hydrazin sowie auch hydroxyalkylierten Phenolen, wie z. B. Di-(β-hydroxyethoxy)-resorcin, hergestellt werden.

Ebenso können hydroxylgruppenhaltige Polyurethane und/oder Polyharnstoffe eingesetzt werden.

Als Polyhydroxylverbindungen können selbstverständlich auch Gemische mehrerer Stoffe eingesetzt werden.

Das Mischungsverhältnis von hydroxylgruppenhaltigen Polymeren und Isocyanatkomponente wird in der Regel so gewählt, daß auf eine OH-Gruppe 0,6 - 1,2, bevorzugt 0,8 - 1,1, ganz besonders bevorzugt 1,0, freie und/oder blockierte NCO-Gruppen kommen.

Die Isocyanatkomponente wird für die Herstellung von PUR-Pulverlacken mit dem geeigneten hydroxylgruppenhaltigen Polymeren und gegebenenfalls Katalysatoren sowie Pigmenten, Füllstoffen und Verlaufmitteln, z. B. Siliconöl, Acrylatharzen, gemischt und in der Schmelze homogenisiert. Dies kann in geeigneten Aggregaten, wie z. B. beheizbaren Knetern, vorzugsweise jedoch durch Exdrudieren, erfolgen, wobei Temperaturobergrenzen von 130 bis 140 °C nicht überschritten werden sollten. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches Pulversprühen, Wirbelsintern, elektrostatisches Wirbelsintern, erfolgen. Nach dem Pulverauftrag werden die beschichteten Werkstücke zur Aushärtung 60 bis 10 Minuten auf eine Temperatur von 160 bis 220 °C, vorzugsweise 30 bis 10 Minuten bei 180 bis 210 °C, erhitzt.

Die Isocyanatkomponente kann zur Herstellung lösemittelhaltiger PUR-Einkomponenten-Einbrennlacke in hierfür geeigneten Lösemitteln gelöst und mit dem für diesen Anwendungsbereich geeigneten hydroxylgruppenhaltigen Polyestern homogenisiert und in bekannter Weise mit den vorgenannten Zuschlagstoffen formuliert werden. Für die erfindungsgemäß hergestellten Einkomponenten-Einbrennlacke geeignete Lösemittel sind solche, deren unterer Siedepunkt bei etwa 100 °C liegt. Die obere Grenze des Siedepunktes des Lösemittels ist von den jeweiligen Einbrenntemperaturen abhängig. Wenn man bei höheren Temperaturen einbrennt, müssen die Siedepunkte der zu verwendenden Lösemittel bei höheren Temperaturen liegen. Als Lösemittel kommen unter anderem folgende infrage: Kohlenwasserstoffe, wie z. B. Toluol, Xylol, SOLVESSO® 100, 150 und 200 (Aromatengemische der Firma Esso), Tetralin, Decalin, Ester, wie z. B. Essigsäurebutylester und -hexylester, Ethylglykolacetat, Butylglykolacetat, Methoxypropylacetat (MOP-A), usw., Ketone, wie z. B. Methylisobutylketon, Diisobutylketon, Isophoron. Die genannten Lösemittel können auch als Gemische eingesetzt werden.

Die PUR-Einkomponenten-Einbrennlacke eignen sich insbesondere für die Applikation auf Metalloberflächen, aber auch auf Gegenständen aus anderen Materialien, wie Glas oder Kunststoff. Die erfindungsgemäß hergestellten Lacke finden auch Anwendung in der Coil-Coating-Lackierung für witterungsbeständige Ein- und Zweischichtlackierungen. Der Auftrag der lösemittelhaltigen, gegebenenfalls pigmentierten Lacksysteme erfolgt durch Rakeln, Rollen, Spritzen, Gießen etc.. Die Härtung der erfindungsgemäßen Einkomponenten-PUR-Einbrennlacke erfolgt - je nach Anwendung - in einem Temperaturbereich von 160 bis 350 °C, vorzugsweise zwischen 180 bis 300 °C, in einer Zeit von 30 Minuten bis 30 Sekunden. Die Lackfilme weisen hervorragende lacktechnische Eigenschaften, insbesondere Flexibilität und Wetterbeständigkeit auf.

Das erfindungsgemäße Verfahren wird nachfolgend anhand von Beispielen beschrieben. Die Dosierung der Reaktionskomponente wird dabei nicht näher behandelt, sie erfolgt mit Hilfe der üblichen Dosierpumpen bzw. Bandwaagen.

### A Herstellung der erfindungsgemäßen Verfahrensprodukte

### Allgemeine Herstellvorschrift

In die Einzugsgehäuse eines Doppelschneckenextruders wurde das aus IPDI hergestellte Uretdion mit einer Temperatur von 60 bis 110 °C eingespeist, wobei gleichzeitig das Diol, das Diolgemisch oder das Diol-Monoalkoholgemisch mit einer Temperatur von 25 bis 110 °C zudosiert wurde. Die OH-Komponente bzw. das Uretdion enthielt ggf. die erforderliche Katalysatormenge, bezogen auf das Endprodukt.

Der eingesetzte Extruder setzt sich aus zehn Gehäusen, die über fünf Heizzonen thermisch gesteuert werden, zusammen. Die Temperaturen der Heizzonen werden wie folgt eingestellt: 1. Zone 60 - 180 °C, 2. Zone 60 - 170 °C, 3. Zone 60 - 150 °C, die 4. Zone 80 - 150 °C und die 5. Zone 70 - 150 °C.

Alle Temperaturen stellen Soll-Temperaturen dar; die Temperatureinstellung in den Knetergehäusen erfolgt durch elektrische Heizung und pneumatische Kühlung. Das Düsenelement wird mittels Ölthermostat beheizt.

Die Drehzahl der Doppelschnecke, die auf der Gesamtlänge aus Förder-und Knetelementen aufgebaut war, beträgt 50 bis 380 Upm.

Das Mengenverhältnis der Reaktanten war dabei so eingestellt, daß das OH/NCO-Verhältnis von 1 : 0,5 bis 1,5, im allgemeinen 1 : 1, beträgt. Das Diol/Monoalkoholgemisch war folgendermaßen eingestellt: 1,75 - 2 : 1.

Das Reaktionsprodukt, das in einer Menge von 10 bis 180 kg/h anfällt, wurde entweder abgekühlt, anschließend zerkleinert oder formiert und abgesackt oder bereits die Schmelze formiert, abgekühlt und abgesackt.

Die physikalischen und chemischen Kenndaten der auf diese Weise erfinderisch hergestellten Beispiele sind in der Tabelle 1 zusammengefaßt.

Die eingesetzten Uretdion-Polyisocyanate hatten folgende Kenndaten:

### IPDI-UD NCO-Gehalt

- frei:: 16,8 - 18,5 Gew.-%
- gesamt:: 37,0 - 37,7 Gew.-%

### HDI-UD NCO-Gehalt

- frei:: 20 - 21,5 Gew.-%
- gesamt:: 35,5 - 37,8 Gew.-%

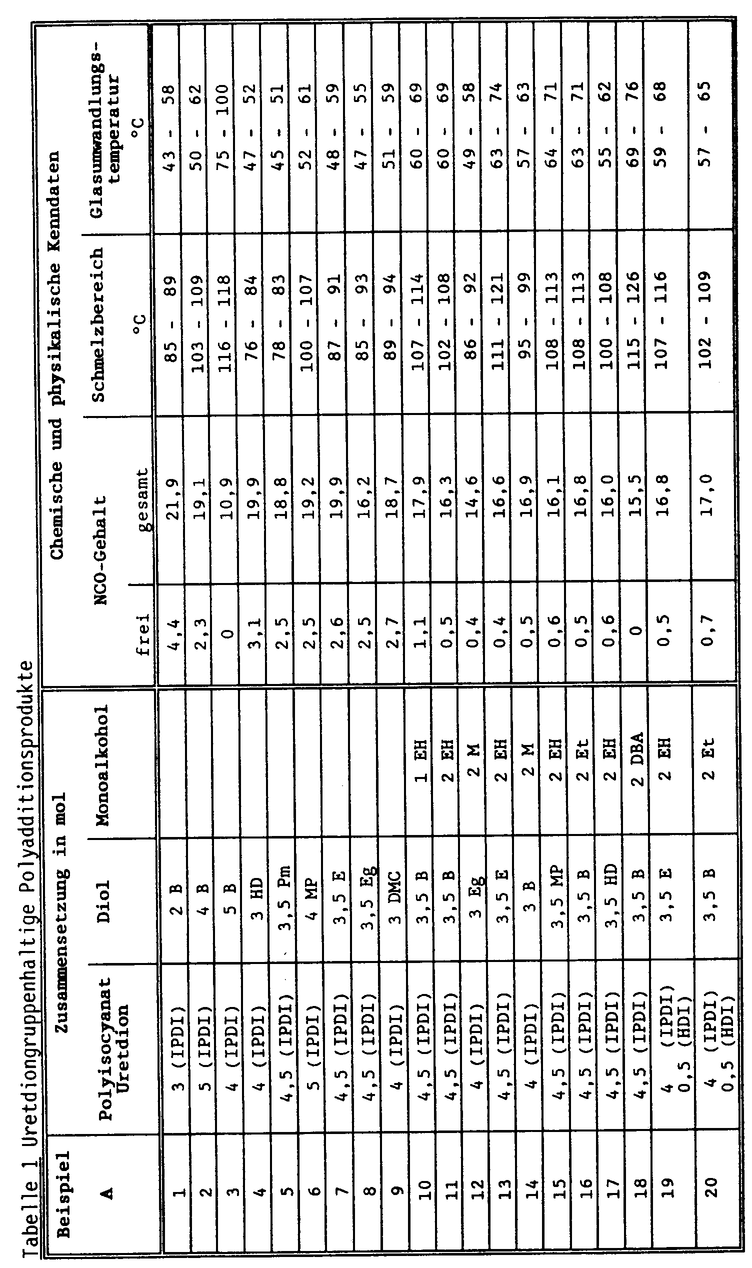

### B Polyolkomponente

### Allgemeine Herstellungsvorschrift

Die Ausgangskomponenten - Terephthalsäure (Ts) und/oder Isophthalsäure (Is) und/oder Dimethylterephthalat (DMT), Hexandiol-1.6 (HD) und/oder Neopentylglykol (NPG) und/oder 1.4-Dimethylolcyclohexan (DMC) und/oder 2.2.4(2.4.4)-Trimethylhexandiol (TMH-d) und Trimethylolpropan (TMP) - werden in einen Reaktor gegeben und mit Hilfe eines Ölbades erwärmt. Nachdem die Stoffe zum größten Teil aufgeschmolzen sind, werden bei einer Temperatur von 160 °C 0,05 Gew.-% Di-n-butylzinnoxid als Katalysator zugesetzt. Die erste Methanolabspaltung tritt bei einer Temperatur von ca. 170 °C auf. Innerhalb 6 bis 8 h wird die Temperatur auf 200 bis 230 °C erhöht und innerhalb weiterer 12 bis 15 h die Reaktion zu Ende geführt. Der Polyester wird auf 200 °C abgekühlt und durch Anlegen von Vakuum (1,33 mbar) innerhalb 30 bis 45 min weitgehend von flüchtigen Anteilen befreit. Während der gesamten Reaktionszeit wird das Sumpfprodukt gerührt und ein schwacher N₂-Strom durch das Reaktionsgemisch geleitet.

Folgende Tabelle gibt die Polyesterzusammensetzungen und die entsprechenden physikalischen und chemischen Kenndaten wieder.

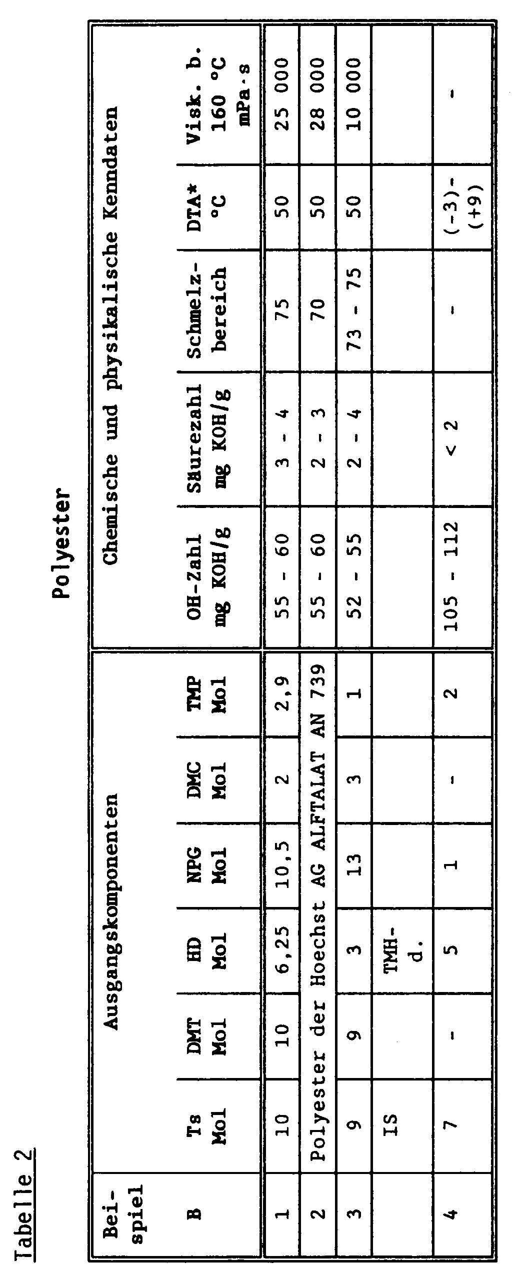

### C Polyurethan-Pulverlacke

### Allgemeine Herstellungsvorschrift

Die gemahlenen Produkte, Vernetzer, Polyester, Verlaufmittel ggf. Katalysator-Masterbatch, werden ggf. mit dem Weißpigment in einem Kollergang innig vermischt und anschließend im Extruder bei 80 bis 120 °C homogenisiert. Nach dem Erkalten wird das Extrudat gebrochen und mit einer Stiftmühle auf eine Korngröße < 100 µm gemahlen. Das so hergestellte Pulver wird mit einer elektrostatischen Pulverspritzanlage bei 60 KV auf entfettete, ggf. vorbehandelte Eisenbleche appliziert und in einem Umlufttrockenschrank bei Temperaturen zwischen 180 und 200 °C eingebrannt.

### Verlaufmittel-Masterbatch

Es werden 10 Gewichtsprozent des Verlaufmittels - ein handelsübliches Copolymer von Butylacrylat und 2-Ethylhexylacrylat - in dem entsprechenden Polyester in der Schmelze homogenisiert und nach dem Erstarren zerkleinert.

### Katalysator-Masterbatch

Es werden 5 Gewichtsprozent des Katalysators - DBTL - in dem entsprechenden Polyester in der Schmelze homogenisiert und nach dem Erstarren zerkleinert.

Die Abkürzungen in den folgenden Tabellen bedeuten:

| | | |
|---|---|---|
| SD | = Schichtdicke in µm | |
| HK | = Härte nach König (sec) | (DIN 53 157) |
| HB | = Härte nach Buchholz | (DIN 53 153) |
| ET | = Tiefung nach Erichsen (mm) | (DIN 53 156) |
| GS | = Gitterschnittprüfung | (DIN 53 151) |
| GG 20 °∢ GG 60 °∢ | = Messung des Glanzes n. Gardner | (ASTM-D 5233) |
| Imp rev. | = Impact reverse in g·m | |

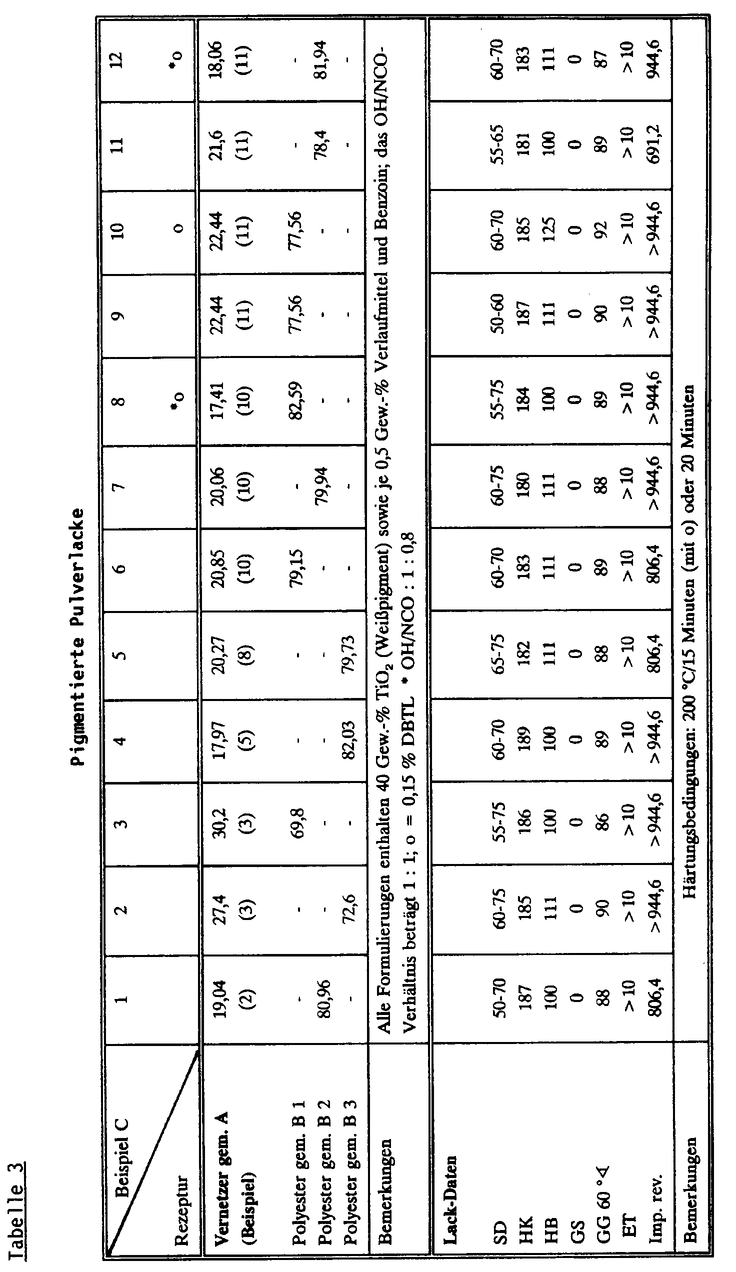

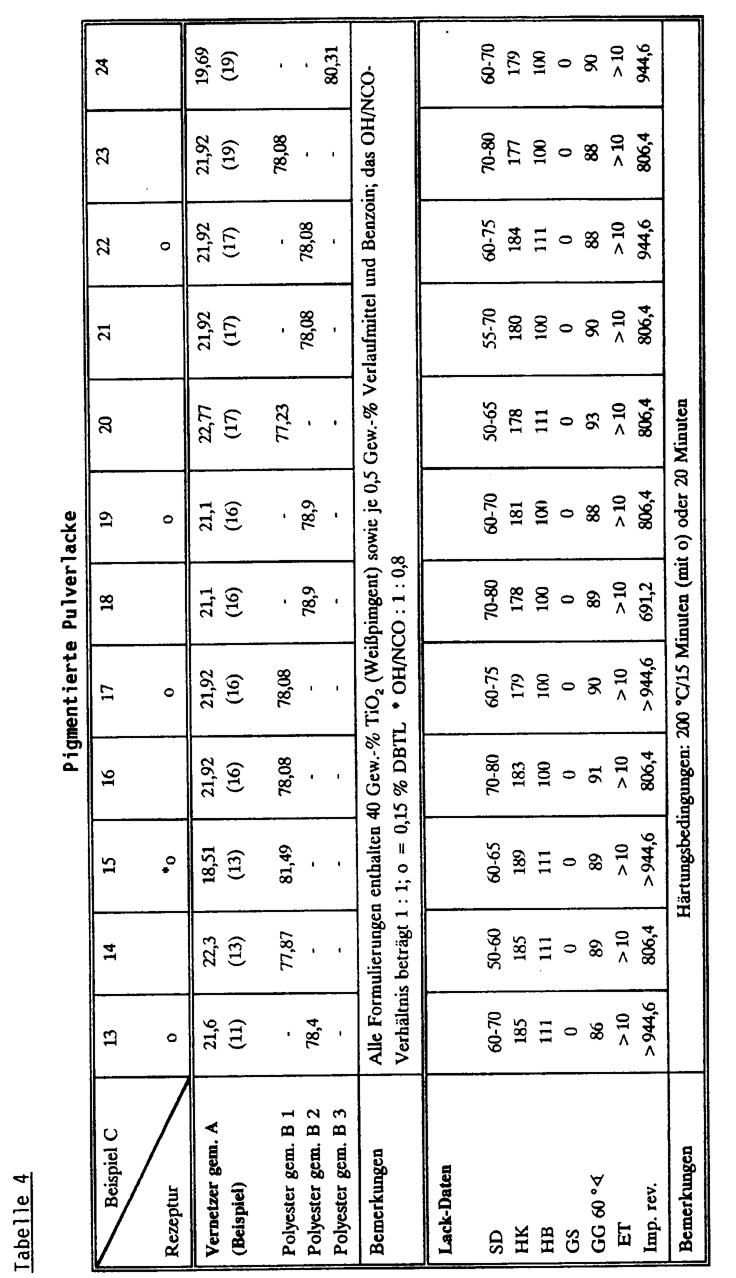

### D Polyurethan-Einkomponenten-Einbrennlacke

### I. Herstellung der Vernetzerlösung

600 Gew.-T. der gemäß A I oder A II hergestellten blockierten Polyisocyanat-Addukte wurden in 400 Gew.-T. Lösemittelgemisch unter Rühren bei 50 - 60 °C gelöst und die Auslaufzeit im DIN-4-Becher bei 20 °C oder im Rotations-Viskosimeter bei 25 °C bestimmt.
a) A 12
   (MOP-A/Solvesso 100 = 1 : 2) 10 500 mPa·s
b) A 3
   (MOP-A/Solvesso 100 = 1 : 2) 22 000 mPa·s
c) A 14
   (MOP-A/Solvesso 100 = 1 : 2) 45 500 mPa·s
d) A 11
   (MOP-A/Solvesso 100 = 1 : 2) 55 000 mPa·s

### II. Herstellung der Polvesterlösung

600 Gew.-T. Polyester gemäß B 4 und B 5 wurden unter Rühren in 400 Gew.-T. Lösemittelgemisch bei 80 - 90 °C gelöst und die Auslaufzeit im DIN-4-Becher bei 20 °C bestimmt.
a) B 4
   (MOP-A/Solvesso 100 = 1 : 2) 410 Sekunden

### III. Herstellung der PUR-Einkomponenten-Einbrennlacke

### a) Lackstammlösung

Die 60 gew.-%ige Polyesterlösung wurde unter Rühren mit der berechneten Menge der 60 gew.-%igen Vernetzerlösung bei 50 - 60 °C homogenisiert und die Auslaufzeit im DIN-4-Becher bei 20 °C bestimmt.
1) gem. Beisp. D I a und gem. Beisp. D II = 370 Sekunden
2) gem. Beisp. D I b und gem. Beisp. D II = 1 150 Sekunden
3) gem. Beisp. D I c und gem. Beisp. D II = 850 Sekunden
4) gem. Beisp. D I d und gem. Beisp. D II = 950 Sekunden

### b) Pigmentierte Lacklösungen

Die gemäß D III a) hergestellten Lackstammlösungen wurden, falls erforderlich, mit weiterem Lösemittelgemisch - entsprechend der Stammlösung - versetzt und anschließend mit Weißpigment (TiO₂) und einem in der PUR-Chemie üblichen Verlaufmittel und Entschäumer in der Kugelrührwerksmühle abgerieben.

Die Applikation dieser pigmentierten Lacke erfolgte auf entfettenen und/oder vorbehandelten 0,8 - 1 mm Stahl- und/oder Aluminiumblechen; die Aushärtung wurde in einem Labor-Umlufttrockenschrank durchgeführt; Härtungstemperaturen lagen zwischen 180 und 250 °C; die Schichtdicke der Lackfilme lag je nach Anwendung zwischen 25 und 50 µm.

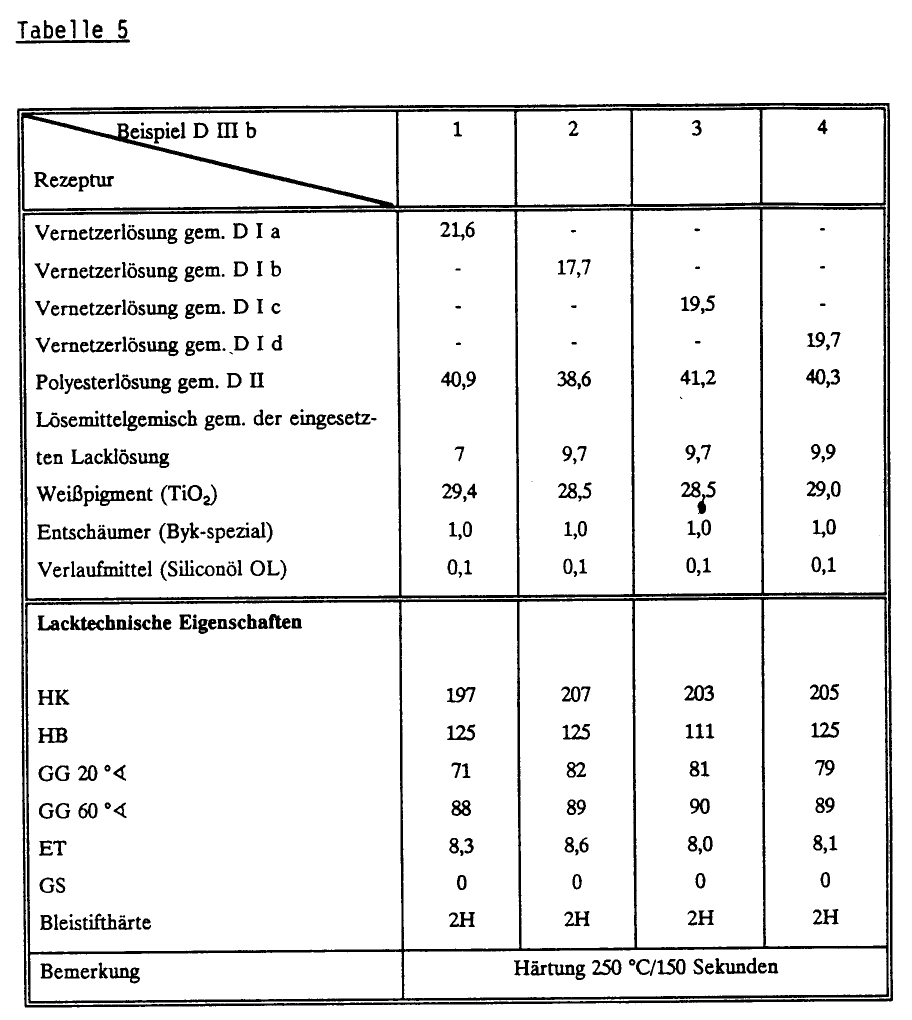

## Patentansprüche

1. Verfahren zur Herstellung von uretdiongruppenhaltigen Polyadditionsprodukten bestehend aus Polyisocyanat-Uretdionen mit mindestens zwei freien Isocyanatgruppen und Diolen, wobei ein OH/NCO-Verhältnis von 0,5 bis 1,5 : 1 zugrunde liegt,
dadurch gekennzeichnet,
daß diese lösemittelfrei und kontinuierlich in einem Intensivkneter in einem Ein- oder Mehrschneckenextruder hergestellt werden.

2. Verfahren nach Anspruch 1,
dadurch gekennzeichnet,
daß die verbleibenden freien NCO-Gruppen partiell oder total mit Monoalkoholen oder Monoaminen umgesetzt werden.

3. Verfahren nach Anspruch 1 bis 2,
dadurch gekennzeichnet,
daß die Reaktion in einem Zweischneckenextruder erfolgt.

4. Verfahren nach den Ansprüchen 1 bis 3,
dadurch gekennzeichnet,
daß die Temperatur im Intensivkneter oder Zweischneckenextruder bis 190 °C beträgt.

5. Verfahren nach den Ansprüchen 1 bis 3,
dadurch gekennzeichnet,
daß die Temperatur im Intensivkneter oder Zweischneckenextruder bis 180 °C beträgt.

6. Verfahren nach den Ansprüchen 1 bis 3,
dadurch gekennzeichnet,
daß die Temperatur im Intensivkneter oder Zweischneckenextruder bis 170 °C beträgt.

7. Verfahren nach den Ansprüchen 1 bis 6,
dadurch gekennzeichnet,
daß die Intensivkneter durch geeignete Bestückung der Mischkammern und Zusammenstellung der Schneckengeometrie einerseits zu einer intensiven raschen Durchmischung und zu hochviskosen Produktströmen bei gleichzeitigem intensiven Wärmeaustausch führen, und andererseits eine gleichmäßige Durchströmung in Längsrichtung mit möglichst einheitlicher Verweilzeit bewirken.

8. Verfahren nach den Ansprüchen 1 bis 7,
dadurch gekennzeichnet,
daß Umsetzungsprodukte und Katalysator in getrennten Produktströmen dem Extruder zugeführt werden.

9. Verfahren nach den Ansprüchen 1 bis 8,
dadurch gekennzeichnet,
daß bei mehr als zwei Produktströmen diese gebündelt zugeführt werden können.

10. Verfahren nach den Ansprüchen 1 bis 9,
dadurch gekennzeichnet,
daß die Diole und/oder Monoalkohole/-amine und/oder Katalysatoren zu einem Produktstrom zusammengefaßt werden können.

11. Verfahren nach den Ansprüchen 1 bis 10,
dadurch gekennzeichnet,
daß die gegenüber dem Polyisocyanat inerten Zuschlagsstoffe gemeinsam mit dem verwendeten Polyisocyanat zu einem Produktstrom zusammengefaßt werden.

12. Verfahren nach den Ansprüchen 1 bis 11,
dadurch gekennzeichnet,
daß die Eintrittsstelle der Produktströme in der Reihenfolge variabel und zeitlich versetzt gehandhabt werden kann.

13. Verfahren nach den Ansprüchen 1 bis 12,
dadurch gekennzeichnet,
daß eine Nachreaktion angefügt werden kann.

14. Verfahren nach den Ansprüchen 1 bis 13,
dadurch gekennzeichnet,
daß die Konfektionierung je nach Viskosität des den Intensiv-Mischer bzw. Extruder und/oder die Nachreaktionszone verlassenden Produkts zunächst durch weitere Abkühlung auf eine zur späteren Absackung/Silierung hinreichende Temperatur eingeleitet wird und der Absackung eine Zerkleinerung vorgeschaltet ist, wobei während des Abkühlens an geeigneter Stelle eine Vorprägung des bevorzugt bandförmig anfallenden Produkts erfolgt, die eine nachfolgende Zerkleinerung in eine gewünschte Partikelgröße/Granulatform vorbereitet und den Staubanfall vermindert.

## Claims

1. A process for the preparation of polyaddition products containing uretdione groups and comprising polyisocyanate-uretdiones having at least two free isocyanate groups and diols, an OH/NCO ratio of from 0.5 to 1.5:1 being used as the basis, characterized in that said polyaddition products are prepared by a solvent-free and continuous procedure in an intensive kneading apparatus in a single- or multiscrew extruder.

2. A process according to claim 1, characterized in that the remaining free NCO groups are reacted in part or in whole with monoalcohols or monoamines.

3. A process according to either of claims 1 and 2, characterized in that the reaction is carried out in a twin-screw extruder.

4. A process according to any of claims 1 to 3, characterized in that the temperature in the intensive kneading apparatus or twin-screw extruder is up to 190°C.

5. A process according to any of claims 1 to 3, characterized in that the temperature in the intensive kneading apparatus or twin-screw extruder is up to 180°C.

6. A process according to any of claims 1 to 3, characterized in that the temperature in the intensive kneading apparatus or twin-screw extruder is up to 170°C.

7. A process according to any of claims 1 to 6, characterized in that the intensive kneading apparatus, by appropriate equipping of the mixing chambers and composition of the screw geometry, on the one hand leads to an intensive, rapid mixing and to highly viscous product streams coupled with intensive heat exchange, and on the other hand effects a uniform flow in the longitudinal direction with a residence time which is as uniform as possible.

8. A process according to any of claims 1 to 7, characterized in that reaction products and catalyst are supplied to the extruder in separate product streams.

9. A process according to any of claims 1 to 8, characterized in that, in the case of more than two product streams, these streams can be supplied in combined form.

10. A process according to any of claims 1 to 9, characterized in that the diols and/or monoalcohols and/or monoamines and/or catalysts can be combined to form one product stream.

11. A process according to any of claims 1 to 10, characterized in that the additives which are inert towards the polyisocyanate are combined with the polyisocyanate used to form one product stream.

12. A process according to any of claims 1 to 11, characterized in that the input of the product streams can be made variable in sequence and offset in terms of time.

13. A process according to any of claims 1 to 12, characterized in that an after-reaction can be appended.

14. A process according to any of claims 1 to 13, characterized in that the final stages of processing are initiated, depending on the viscosity of the product leaving the intensive mixer or extruder and/or the after-reaction zone, first by further cooling to a temperature which is adequate for subsequent bag-filling/containerization, with comminution being carried out before bag-filling, in which process, during cooling, at an appropriate point, the product which is produced preferably in strip form is pre-impressed in order to prepare for subsequent comminution to a desired particle size/granule form, and to reduce the amount of dust obtained.

## Revendications

1. Procédé de fabrication de produits de polyaddition contenant des groupes uretdione, constitués de polyisocyanate/uretdiones avec au moins deux groupes isocyanates libres et de diols, qui reposent sur un rapport OH/NCO de 0,5 à 1,5 : 1
caractérisé en ce que
ceux-ci sont préparés sans solvant et en continu dans un malaxeur intensif ou dans une extrudeuse à une ou plusieurs vis.

2. Procédé selon la revendication 1,
caractérisé en ce que
les groupes NCO libres, qui demeurent, sont mis à réagir partiellement ou totalement avec des monoalcools.

3. Procédé selon la revendication 1 à 2,
caractérisé en ce que
la réaction s'effectue dans une extrudeuse à deux vis.

4. Procédé selon les revendications 1 à 3,
caractérisé en ce que
la température dans le malaxeur intensif ou dans l'extrudeuse à deux vis s'élève jusqu'à 190°C.

5. Procédé selon la revendication 1 à 3,
caractérisé an ce que
la température dans le malaxeur intensif ou dans l'extrudeuse à deux vis s'élève jusqu'à 180°C.

6. Procédé selon la revendication 1 à 3,
caractérisé en ce que
la température dans le malaxeur intensif ou dans l'extrudeuse à deux vis s'élève jusqu'à 170°C.

7. Procédé selon les revendications 1 à 6,
caractérisé en ce que
les malaxeurs intensifs conduisent par équipement approprié des chambres de mélange et réunion de géométrie des vis, d'une part, à un mélange intensif rapide et à des courants de produits de viscosité élevée avec un échange de chaleur intensif simultané et, d'autre part, entraînent une traversée homogène dans la direction longitudinale avec un temps de séjour le plus uniforme possible.

8. Procédé selon les revendications 1 à 7,
caractérisé en ce que
les produits de réaction et le catalyseur sont amenés à l'extrudeuae dans des courants de produits séparés.

9. Procédé selon les revendications 1 à 8,
caractérisé en ce que
pour plus de deux courants de produits, ceux-ci peuvent être amenés liés ensemble.

10. Procédé selon les revendications 1 à 9,
caractérisé en ce que
les diols et/ou les monoalcools ou amines et/ou les catalyseurs peuvent être réunis en un courant de produits.

11. Procédé selon les revendications 1 à 10,
caractérisé en ce que
les substances additionnelles, inertes vis-à-vis des polylsocyanates, sont réunies conjointement au polyisocyanate utilisé, en un courant de produits.

12. Procédé selon les revendications 1 à 11,
caractérisé en ce que
l'emplacement d'entrée des courants de produits peut être manoeuvré dans la séquence d'une manière variable et déplacée dans le temps.

13. Procédé selon les revendications 1 à 12,
caractérisé et ce que
on peut ajouter une réaction ultérieure.

14. Procédé selon les revendications 1 à 13,
caractérisé en ce que
le conditionnement selon la viscosité du produit qui quitte le mélangeur intensif ou l'extrudeuse et/ou la zone de réaction ultérieure est induit et premier lieu grâce à un refroidissement supplémentaire à une température suffisante pour un ensachage ou à une mise en silo ultérieure, et on connecte, avant l'ensachage, un broyage comprenant, pendant le refroidissement à un endroit approprié, un pré-estampage du produit qui se présente de préférence sous forme de bande, pré-estampage qui prépare un broyage subséquent à une taille de particules ou sous forme de granulé désirée, et qui diminue l'émission de poussières.
